Europäisches Patentamt

European Patent Office

Office européen des brevets

(11) Veröffentlichungsnummer: **0 330 094 B1**

(12) **EUROPÄISCHE PATENTSCHRIFT**

(45) Veröffentlichungstag der Patentschrift: **04.11.92**

(51) Int. Cl.⁵: **A61K 35/78**

(21) Anmeldenummer: **89102796.3**

(22) Anmeldetag: **20.02.89**

(54) **Verwendung der Pflanze Euphorbia hirta L. und ihrer Extrakte sowie ihrer Wirkstoffe.**

(30) Priorität: **25.02.88 DE 3805965**

(43) Veröffentlichungstag der Anmeldung:
**30.08.89 Patentblatt 89/35**

(45) Bekanntmachung des Hinweises auf die
Patenterteilung:
**04.11.92 Patentblatt 92/45**

(84) Benannte Vertragsstaaten:
**AT BE CH DE ES FR GB IT LI LU NL SE**

(56) Entgegenhaltungen:

BIOLOGICAL ABSTRACTS, Band 72, 1981, Nr.
4172, Philadelphia, US; W.I. HWANG et al.:
"Extraction of anticancer components from
Korean medicinal plants and the determination of their cytotoxic activities on the cancer cells", & KOREAN BIOCHEM J 13(1),
25-40, 1980

BIOLOGICAL ABSTRACTS, Band 72, 1981, Nr.
18542, Philadelphia, US; G.C. HOKANSON et
al.: "Dry column chromatographic procedure
for rapid concentration of biological activity
in natural products fractionation", & J
PHARM SCI 70(3), 329-331, 1981

BIOLOGICAL ABSTRACTS, Band 81, 1986, Nr.
75056, Philadelphia, US; A.O. AJAO et al.:
"Antibacterial activity of Euphorbia hirta", &
FITOTERAPIA 56(3), 165-167, 1985

BIOLOGICAL ABSTRACTS, Band 69, 1980, Nr.
18145, Philadelphia, US; M.M. EL-MERZABANI
et al.: "Screening system for Egyptian plants
with potential antitumor activity", & PLANTA
MED 36(2): 150-155, 1979

(73) Patentinhaber: **Tamás, Eszter, geb. Szénási**
**c/o Hompothnè Szènàsi Laura Vàròshàz Tèr**
**19**
**H-7300 K o m l ò(HU)**

(72) Erfinder: **Tamás, Eszter, geb. Szénási**
**c/o Hompothnè Szènàsi Laura Vàròshàz Tèr**
**19**
**H-7300 K o m l ò(HU)**

(74) Vertreter: **Beszédes, Stephan G., Dr. Patent-**
**anwalt**
**Münchener Strasse 80a Postfach 1168**
**W-8060 Dachau(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

Die Erfindung betrifft die Verwendung von wäßrigen oder wäßrig-alkoholischen Extrakten der Pflanze Euphorbia hirta L. sowie ihrer aus diesen erhaltenen Wirkstoffe.

Es ist bereits bekannt, die Pflanze Euphorbia hirta L., welche der Familie der Euphorbiaceae angehört, gegen Amibiase, Verminose und Diarrhoe zu verwenden.

Ferner ist aus Journal of Pharmaceutical Sciences, Vol. 70, No. 3, 1981, Seiten 329 bis 331 (referiert in Biological Abstracts, Band 72, 1981, Nr. 18542) eine gegen Tumoren wirksame Chloroform-Fraktion eines Extraktes aus Euphorbia cyparissias beschrieben. Die wäßrige Fraktion wurde als offensichtlich unwirksam unbeachtet gelassen.

Der Erfindung liegt die Aufgabe zugrunde, die Verwendung von Extrakten einer Pflanze und ihrer aus diesen erhaltenen Wirkstoffe zur Bekämpfung von Tumoren und Geschwulsten zu schaffen.

Das Obige wurde überraschenderweise durch die Erfindung erreicht.

Es wurde nämlich überraschenderweise festgestellt, daß die wäßrigen oder wäßrig-alkoholischen Extrakte der Pflanze Euphorbia hirta L. und ihrer aus diesen erhaltenen Wirkstoffe eine hervorragende Wirkung als tumor- und geschwulsthemmende Mittel haben.

Gegenstand der Erfindung ist daher die Verwendung von wäßrigen oder wäßrig-alkoholischen Extrakten der Pflanze Euphorbia hirta L. sowie ihrer aus diesen erhaltenen Wirkstoffe zur Herstellung von Arzneimitteln zur Tumor- und Geschwulstbekämpfung.

Diese Pflanze kommt in tropischen und subtropischen Gegenden vor.

Erfindungsgemäß können die Extrakte der ganzen Pflanzen beziehungsweise ihre aus diesen erhaltenen Wirkstoffe verwendet werden. Nach einer zweckmäßigen Ausführungsform werden nur die Extrakte der Stengel und Blätter beziehungsweise ihre aus diesen erhaltenen Wirkstoffe eingesetzt.

Vorteilhaft werden zur Herstellung der Extrakte 5 bis 50 g, Vorzugsweise 10 bis 20 g, getrocknete Pflanze beziehungsweise Pflanzenteile je 1 Extraktionsmittel, wie Wasser, verwendet.

Zweckmäßig wird die Extraktion mit siedendem Extraktionsmittel, wie siedendem Wasser, durchgeführt.

Die Isolierung der Wirkstoffe kann nach üblichen Verfahren durchgeführt werden.

Die Extrakte der Pflanze Euphorbia hirta L. und ihrer Teile beziehungsweise die aus diesen erhaltenen Wirkstoffe können in Form von üblichen flüssigen und festen Arzneimittelpräparaten, beispielsweise Tees, Tinkturen, Lösungen, Suspensionen, Kapseln, Pillen, Suppositorien und Injektionslösungen erfindungsgemäß verwendet werden. Sie können aber auch als solche eingesetzt werden.

Die Erfindung umfaßt daher auch die Verwendung der genannten Extrakte der Pflanze Euphorbia hirta L. sowie ihrer aus diesen erhaltenen Wirkstoffe zur Herstellung von Arzneimitteln.

Nach einer zweckmäßigen Ausführungsform der erfindungsgemäßen Verwendung wird ein Tee aus der Pflanze Euphorbia hirta L. und/oder Teilen derselben eingesetzt.

Die erfindungsgemäße Verwendung umfaßt die Bekämpfung sämtlicher Tumore, und zwar sowohl krebsartiger als auch nicht krebsartiger. Als besonders hervorragend hat sich die erfindungsgemäße Verwendung gegen Brustkrebs, Gebärmutterkrebs beziehungsweise -geschwulst und Magenkrebs beziehungsweise -geschwulst. Weitere Beispiele für die erfindungsgemäße Verwendung sind die gegen Gehirntumore, Knochenschwundtumore, Nierentumore und auch allgemein Tumore mit Metastasen.

Die zweckmäßige Dosis bei der erfindungsgemäßen Verwendung ist wie folgt: Mit 0,1 1 heißem Wasser aus 1 bis 2 g getrockneter Pflanze beziehungswieise getrockneten Pflanzenteilen gewonnener Extrakt beziehungsweise daraus hergestellte Wirkstoffe. Die Verabreichungshäufigkeit beträgt zweckmäßig 2-mal täglich.

Beispiele für die erfolgreiche erfindungsgemäße Verwendung sind wie folgt:

Fall A: 5 Jahre nach der Operation wegen Brustkrebs bei einer etwa 50-jährigen Frau erneuerte sich dieser wieder. 10 Tage nach der erfindungsgemäßen Verwendung in Teeform trat eine völlige Genesung, die anhielt, ein.

Fall B: Eine Gebärmuttergeschwulst (von welcher es nicht bekannt war, ob sie krebsartig ist) verschwand auf die erfindungsgemäße Verwendung in Teeform hin völlig.

Fall C: Bei einer 50-jährigen Frau mit einer Brustgeschwulst nach Operation und darauf folgende Entfernung der Gebärmutter und des Eierstockes und nach Erscheinen der Geschwulste auf der anderen Brust verschwanden alle Geschwulste und kamen nicht mehr wieder.

Fall D: Bei einer 58-jährigen Frau mit Magenkrebs, die bereits Atmungsstörungen hatte, wurde der Magen zur Operation aufgeschnitten, jedoch als nicht operierbar wieder zugenäht. Nach 5-tägiger erfindungsgemäßer Verwendung in Teeform konnte diese Frau bereits essen, es ging ihr zunehmend besser und sie kann nach einigen Monaten wieder alles essen.

Fall E: Bei einer 42-jährigen Frau mit Brust- und Nierengeschwulst, die bestrahlt wurde und deren Urin nurmehr mittels Katheter entfernbar war, konnte nach 4-tägiger erfindungsgemäßer Verwendung in Teeform der Katheter entfernt werden und nach 8-tägiger Verwendung verschwanden die Geschwulste von ihrer Brust.

Fall F: Bei einer 38-jährigen Frau wurde die eine Brust wegen einer krebsartigen Geschwulst entfernt und sie wurde bestrahlt. Danach ging die Geschwulst auf die Wirbelsäule über. Nach 1-wöchiger erfindungsgemäßer Verwendung in Teeform verschwand ihre Wirbelsäulengeschwulst und ihr Blutbild wurde gut.

Fall G.: Eine 40-jährige Frau hatte Magenschmerzen, sie konnte nicht essen und nahm plötzlich um 50 kg ab, so daß die Ärzte sie aufgaben. Nach 1-wöchiger erfindungsgemäßer Verwendung konnte sie alles essen und ihr Gewicht nahm zu.

Die Herstellung der erfindungsgemäß verwendeten Extrakte wird an Hand des folgenden Beispieles näher erläutert.

Beispiel

Es wurde die ganze Pflanze Euphorbia hirta L. an der Luft getrocknet. Ferner wurde Wasser aufgekocht. Je 1 bis 2 g der getrockneten Pflanze wurden in 0,1 l des kochenden Wassers eingebracht und darin ziehen gelassen. Dann wurde abkühlen gelassen und abfiltriert. Der als Filtrat erhaltene Tee war gebrauchsfertig.

Zweckmäßig wurde der wie vorstehend beschrieben mit 0,1 l Wasser hergestellte Extrakt von 1 bis 2 g Pflanze 2-mal täglich getrunken.

## Patentansprüche

1. Verwendung von wäßrigen oder wäßrig-alkoholischen Extrakten der Pflanze Euphorbia hirta L. sowie ihrer aus diesen erhaltenen Wirkstoffe zur Herstellung von Arzneimitteln zur Tumor- und Geschwulstbekämpfung.

2. Verwendung nach Anspruch 1, dadurch gekennzeichnet, daß man einen Tee aus der Pflanze Euphorbia hirta L. und/oder Teilen derselben einsetzt.

## Claims

1. The use of aqueous or aqueous-alcoholic extracts of the plant Euphorbia hirta L. as well as of its active principles obtained from them for the preparation of medicaments for combatting tumours or ulcers.

2. The use according to claim 1, characterized by that one applies a tea from the plant Euphorbia hirta L. and/or parts of it.

## Revendications

1. Utilisation de extraits aqueux ou aqueux-alcooliques de la plante Euphorbia hirta L. ainsi que de ses principes actifs obtenus en ces pour combattre tumeurs et ulcères.

2. Utilisation selon la revendication 1, caractérisé en ce que on emploie un thé de la plante Euphorbia hirta L. et/ou de ses parties.